# EUROPÄISCHE PATENTSCHRIFT

(11) **EP 0 689 418 B1**
(45) Veröffentlichungstag und Bekanntmachung des Hinweises auf die Patenterteilung: **30.09.1998**
(21) Anmeldenummer: 94909109.4
(22) Anmeldetag: 03.03.1994
(51) Int. Cl.: A61K 7/32

(54) **DESODORIERENDE WIRKSTOFFKOMBINATIONEN AUF DER BASIS VON WOLLWACHSSÄUREN UND PARTIALGLYCERIDEN**
DEODORANT ACTIVE-SUBSTANCE COMBINATIONS MADE FROM WOOL-GREASE ACIDS AND PARTIAL GLYCERIDES
COMBINAISONS DE SUBSTANCES ACTIVES DEODORANTES A BASE D'ACIDES DE LANOLINE ANHYDRE ET DE GLYCERIDES PARTIELS

(30) Priorität: 23.03.1993 DE 4309372
(43) Veröffentlichungstag der Anmeldung: 03.01.1996
(73) Patentinhaber: Solvay Alkali GmbH, 30173 Hannover (DE)
(72) Erfinder: KLIER, Manfred, D-20521 Aumühle (DE); SCHNEIDER, Günther, D-20251 Hamburg (DE); TRAUPE, Bernd, D-22457 Hamburg (DE); VOSS, Ilona, D-22549 Hamburg (DE); WOLF, Florian, D-20251 Hamburg (DE); SIEMANOWSKI, Werner, D-47495 Rheinberg (DE); UHLIG, Karl-Heinz, D-47802 Krefeld (DE); RÖCKL, Manfred, D-22880 Wedel/Holst (DE)
(74) Vertreter: Lauer, Dieter, Dr.
(86) Internationale Anmeldenummer: EP9400623
(87) Internationale Veröffentlichungsnummer: WO9421220

(56) Entgegenhaltungen:
- DE-A- 3 320 304
- DE-A- 3 930 193
- AUSTRALIAN JOURNAL OF CHEMISTRY Bd. 24 , 1971 , MELBOURNE (AUSTRALIA) Seiten 153 - 159 B.S. GOODRICH 'ANTIMICROBIAL FACTORS IN WOOL WAX' in der Anmeldung erwähnt

## Beschreibung

Die vorliegende Erfindung betrifft kosmetische Wirkstoffkombinationen, insbesondere Wirkstoffkombinationen als wirksames Prinzip in kosmetischen Desodorantien.

Kosmetische Desodorantien dienen dazu, Körpergeruch zu beseitigen, der entsteht, wenn der an sich geruchlose frische Schweiß durch Mikroorganismen Zersetzt wird. Den handelsüblichen kosmetischen Desodorantien liegen unterschiedliche Wirkprinzipien Zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
4) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und darin liegt die Lösung all dieser Aufgaben, daß kosmetische Desodorantien, enthaltend Gemische aus
I) Wollwachssäuren oder Wollwachssäurekomponenten und
11) Fettsäurepartialglyceriden unverzweigter Fettsäuren, gewählt aus der Gruppe der
   (1) Monocarbonsäure-monoester des Diglycerins der Strukturen 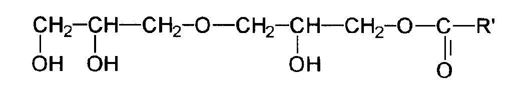 wobei R' einen unverzweigten Alkylrest von 5 bis 17 C-Atomen darstellt,
   (2) Monocarbonsäure-monoester des Triglycerins der Strukturen 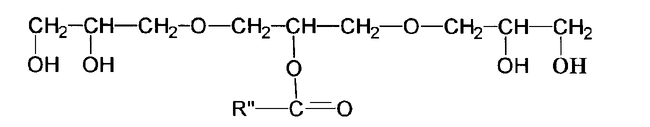 wobei R" einen unverzweigten Alkylrest von 5 bis 17 C-Atomen darstellt,

   den Nachteilen des Standes der Technik abhelfen.

Zwar beschreibt DE-A- 33 20 304 ein Deodorant mit einem Gehalt an Wollwachssäuren und Mono- und Diglyceriden höherer Fettsäuren, konnte aber nicht den Weg zur vorliegenden Erfindung weisen.

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel 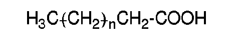
(b) a-Hydroxycarbonsäuren, gemäß der Formel 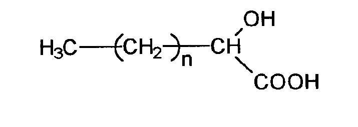
(c) ω-Hydroxycarbonsäuren, gemäß der Formel 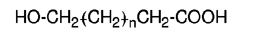
(d) Isocarbonsäuren, gemäß der Formel 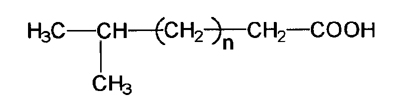
(e) a-Hydroxy-isocarbonsäuren, gemäß der Formel 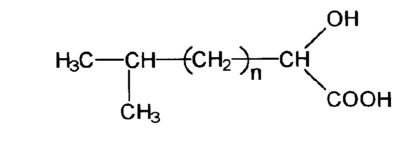
(f) ω-Hydroxy-isocarbonsäuren, gemäß der Formel 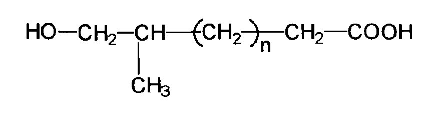
(g) Anteisocarbonsäuren, gemäß der Formel 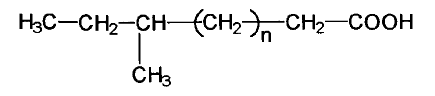
(h) a-Hydroxy-anteisocarbonsäuren, gemäß der Formel 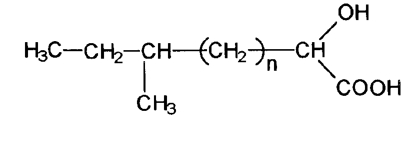
(i) ω-Hydroxy-anteisocarbonsäuren, gemäß der Formel

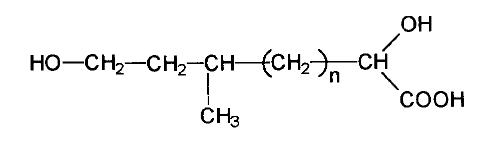

Dabei nimmt n gewöhnlich Werte von 7 - 31 an. Repräsentative Zusammensetzungen der Wollwachssäuren werden beispielsweise in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3.Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Typischerweise bestehen Wollwachssäuren aus ca. 60 % gesättigten, unsubstituierten Carbonsäuren, ca. 30 % a-Hydroxycarbonsäuren und ca. 5 % m-Hydroxycarbonsäuren, wobei der Rest von ca. 5 % im wesentlichen von den anderen vorgenannten Carbonsäuretypen gebildet wird.

Insbesondere zeichnen sich die erfindungsgemäßen Wollwachssäuren vorteilhaft durch folgende kennzeichnende Parameter aus:

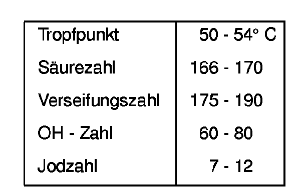

Zwar ist aus dem Aufsatz "Antimicrobial Factors in Wool Wax" (Australian Journal of Chemistry, 1971, 24, Seiten 153 ff. ) bekannt, daß in manchen Wollwachschargen antimikrobielle Faktoren enthalten sind. Ein Hinweis in Richtung der vorliegenden Erfindung findet sich am angegebenen Orte jedoch nicht.

Es wird angenommen, daß insbesondere die a-Hydroxycarbonsäuren einen wesentlichen Beitrag zur erfindinngsgemäßen Wirkung leisten. Erfindungsgemäß sind daher auch kosmetische Desodorantien, enthaltend Gemische aus
I)
   - a-Hydroxycarbonsäuren, gemäß der Formel 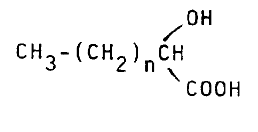 und/oder
   - a-Hydroxy-isocarbonsäuren, gemäß der Formel 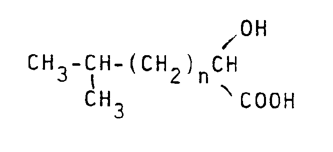 und/oder
   - a-Hydroxy-anteisocarbonsäuren, gemäß der Formel 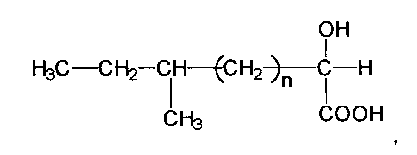 wobei jeweils n Werte von 7 - 31 annimmt, und
11) Fettsäurepartialglyceriden unverzweigter Fettsäuren, gewählt aus der Gruppe der
   (1) Monocarbonsäure-monoester des Diglycerins der Strukturen 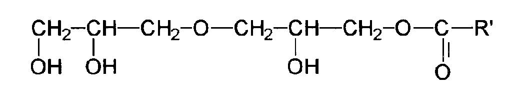 wobei R' einen unverzweigten Alkylrest von 5 bis 17 C-Atomen darstellt,
   (2) Monocarbonsäure-monoester des Triglycerins der Strukturen 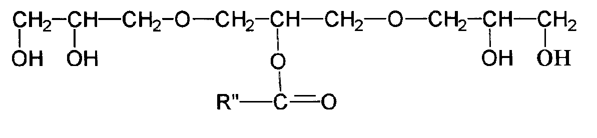 wobei R" einen unverzweigten Alkylrest von 5 bis 17 C-Atomen darstellt

Besonders vorteilhaft im Sinne der vorliegenden Erfindung ist, a-Hydroxycarbonsäuren zu verwenden, welche C₁₆-Körper darstellen, die also am a-Kohlenstoffatom eine verzweigte oder unverzweigte C14H29-Kette tragen.

Vorteilhaft ist weiter, Wollwachssäuregemische zu verwenden, in welchen der Gehalt an a-Hydroxycarbonsäuren 20 - 30 Gew.-%, bezogen auf die Gesamtzusammensetzung beträgt.

Partialglyceride im Sinne der vorliegenden Erfindung sind:
(1) Monocarbonsäure-monoester des Diglycerins.
(2) Monocarbonsäure-monoester des Triglycerins.

Erfindungsgemäß liegen die Di- bzw. Triglycerineinheiten der erfindungsgemäßen Partialglyceride als lineare, unverzweigte Moleküle, also über die jeweiligen OH-Gruppen in 1 - bzw. 3-Stellung veretherte "Monoglycerinmoleküle" vor.

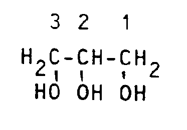

### (Glycerin = "Monoglycerin")

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Partialglyceride auf Basis des Diglycerins sind vorzugsweise Monocarbonsäuremonoester und bevorzugt durch folgende Struktur gekennzeichnet: 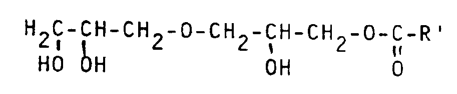 wobei R' einen unverzweigten Alkylrest von 5 bis 17 C-Atomen darstellt.

Die erfindungsgemäßen Partialglyceride auf Basis des Triglycerins sind vorzugsweise Monocarbonsäuremonoester und bevorzugt durch folgende Struktur gekennzeichnet: 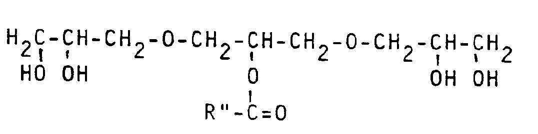 wobei R" einen unverzweigten Alkylrest von 5 bis 17 C-Atomen darstellt.

Die diesen Estern zugrundeliegenden Fettsäuren bzw. Monocarbonsäuren sind die
Hexansäure (Capronsäure) (R' bzw. R" = -C₅H₁₁),
Heptansäure (Önanthsäure) (R' bzw. R" -C₆H₁₃),
Octansäure (Caprylsäure) (R' bzw. R" = -C₇H₁₅),
Nonansäure (Pelargonsäure) (R' bzw. R" = -C₈H₁₇),
Decansäure (Caprinsäure) (R' bzw. R" = -C₉H₁₉),
Undecansäure (R' bzw. R" -C₁₀H₂₁),
10-Undecensäure (Undecylensäure) (R' bzw. R" = -C₁₀H₁₉)_{'}
Dodecansäure (Laurinsäure) (R' bzw. R" = -C₁ H₂₃),
Tridecansäure (R' bzw. R" = -C₁₂H₂₅),
Tetradecansäure (Myristinsäure) (R' bzw. R" = -C₁₃H₂₇),
Pentadecansäure (R' bzw. R" = -Cᵢ₄H₂₉),
Hexadecansäure (Palmitinsäure) (R' bzw. R" = -C₁₅H₃₁),
Heptadecansäure (Margarinsäure) (R' bzw. R" = -C₁₆H₃₃),
Octadecansäure (Stearinsäure) (R' bzw. R" = -C₁₇H₃₅).

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Ester des Diglycerins gegenüber denen des Triglycerins erfindungsgemäß bevorzugt.

Ganz besonders günstig sind
Diglycerinmonocaprinat (DMC) R' = 9
Triglycerinmonolaurat (TML) R" = 11
Diglycerinmonolaurat (DML) R' = 11
Triglycerinmonomyristat (TMM) R" = 13.

Als bevorzugtes erfindungsgemäßes Partialglycerid hat sich das Diglycerinmonocaprinat (DMC) erwiesen.

Insbesondere vorteilhaft sind Partialglyceride auf Basis solcher Monocarbonsäureester, welche nach einem Verfahren erhältlich sind, wie es in der DE-OS 38 18 293 beschrieben wird.

Nach einer vorteilhaften Ausführungsform der vorliegenden Erfindung werden als Partialglyceride unverzweigter Fettsäuren Gemische eines oder mehrerer Monocarbonsäureester des Diglycerins mit einem oder mehreren Monocarbonsäureestern des Triglycerins verwendet.

Nach einer weiteren vorteilhaften Ausführungsform der vorliegenden Erfindung werden als Partialglyceride unverzweigter Fettsäuren ein oder mehrere Monocarbonsäureester des Diglycerins und/oder ein oder mehrere Monocarbonsäureester des Triglycerins in Kombination mit anderen in Kosmetika üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf 100 Gew.-Teile der Gesamtmenge, welche sich aus dem Monocarbonsäureester bzw. den Monocarbonsäureestern des Diglycerins und/oder des Triglycerins und diesen Ersatzwirkstoffen und/oder Verschnittstoffen zusammensetzt.

Nach einer weiteren vorteilhaften Ausführungsform der vorliegenden Erfindung werden als Wollwachssäurekomponenten ein oder mehrere
(a) gesättigte unsubstitinierte Carbonsäuren, gemäß der Formel 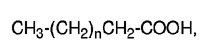 und/oder
(b) a-Hydroxycarbonsäuren, gemäß der Formel 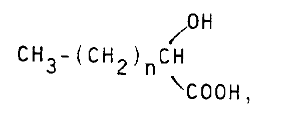 und/oder
(c) n-Hydroxycarbonsäuren, gemäß der Formel 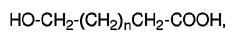 und/oder
(d) Isocarbonsäuren, gemäß der Formel 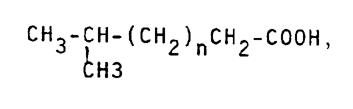 und/oder
(e) a-Hydroxy-isocarbonsäuren, gemäß der Formel 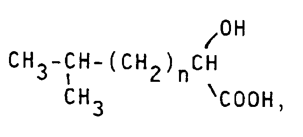 und/oder
(f) Ω-Hydroxy-isocarbonsäuren, gemäß der Formel 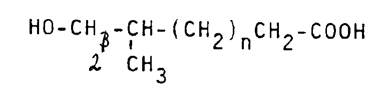 und/oder
(g) Anteisocarbonsäuren, gemäß der Formel 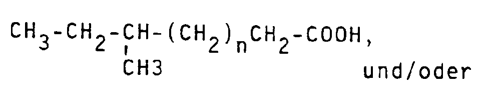
(h) a-Hydroxy-anteisocarbonsäuren, gemäß der Formel 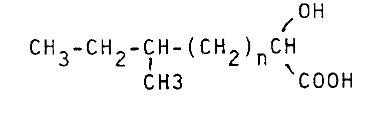 und/oder
(i) Ω-Hydroxy-anteisocarbonsäuren, gemäß der Formel 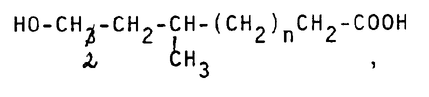 wobei n Werte von 7 - 31 annimmt, in Kombination mit anderen in Kosmetika üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf 100 Gew.-Teile der Gesamtmenge, welche sich aus der Summe der Wollwachssäurekomponenten und diesen Ersatzwirkstoffen und/oder Verschnittstoffen zusammensetzt.

Als besonders vorteilhafte Verkörperung der vorliegenden Erfindung werden kosmetische Desodorantien mit einem Gehalt an
I) Wollwachssäuren bzw. Wollwachssäurekomponenten und
II) einem oder mehreren Substanzen gewählt aus der Gruppe
   Diglycerinmonocaprinat (DMC) ,
   Triglycerinmonolaurat (TML) ,
   Diglycerinmonolaurat (DML) ,
   Triglycerinmonomyristat (TMM)

   angesehen.

Vorteilhafte Verkörperungen der vorliegenden Erfindung stellen auch kosmetische Desodorantien mit einem Gehalt an
I)
   - a-Hydroxycarbonsäuren, gemäß der Formel 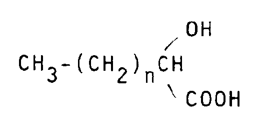 und/oder
   - a-Hydroxy-isocarbonsäuren, gemäß der Formel 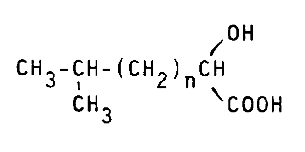 und/oder
   - a-Hydroxy-anteiosocarbonsäuren, gemäß der Formel 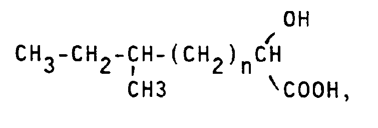 wobei n jeweils eine Zahl von 7 bis 31 darstellt, und
I
   Diglycerinmonocaprinat (DMC) und/oder
   Triglycerinmonolaurat (TML) und/oder
   Diglycerinmonolaurat (DML) und/oder
   Triglycerinmonomyristat (TMM)

   dar.

Es ist von Vorteil, den Gehalt an
I) Wollwachssäuren bzw. Wollwachssäurekomponenten und
II) Fettsäurepartialglyceriden unverzweigter Fettsäuren

so zu wählen, daß Verhältnisse von I) und II) wie 5 : 1 bis 1 : 5, insbesondere wie etwa 1 : 1, ganz besonders vorteilhaft wie etwa 1 : 3 entstehen.

Erfindungsgemäß ist ferner ein Verfahren zur Bekämpfung des durch mikrobielle Zersetzung apokrinen Schwei ßes hervorgerufenen menschlichen Körpergeruches, dadurch gekennzeichnet, daß eine wirksame Menge an Gemischen aus
I) Wollwachssäuren oder Wollwachssäurekomponenten und
II) Fettsäurepartialglyceriden unverzweigter Fettsäuren

insbesondere

Gemischen aus
I)
   - a-Hydroxycarbonsäuren, gemäß der Formel 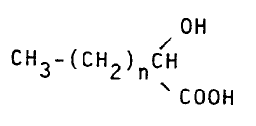 und/oder
   - a-Hydroxy-isocarbonsäuren, gemäß der Formel 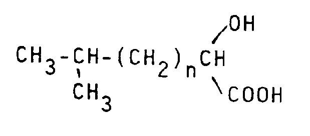 und/oder
   - a-Hydroxy-anteiosocarbonsäuren, gemäß der Formel 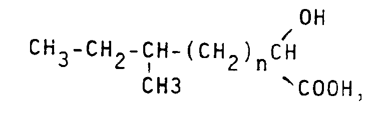 wobei n jeweils eine Zahl von 7 bis 31 darstellt, und
II Fettsäurepartialglyceriden unverzweigter Fettsäuren, welche gegebenenfalls in einem geeigneten kosmetischen Träger vorliegen können, auf die Haut aufgetragen wird.

Erfindungsgemäß ist schließlich auch die Verwendung von Gemischen aus
I) Wollwachssäuren oder Wollwachssäurekomponenten und
II) Fettsäurepartialglyceriden unverzweigter Fettsäuren,

insbesondere Gemischen aus
I)
   - a-Hydroxycarbonsäuren, gemäß der Formel 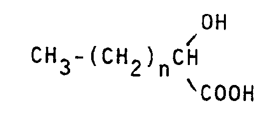 und/oder
   - a-Hydroxy-isocarbonsäuren, gemäß der Formel 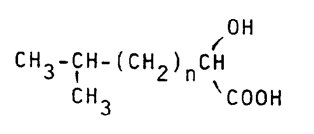 und/oder
   - a-Hydroxy-anteisocarbonsäuren, gemäß der Formel 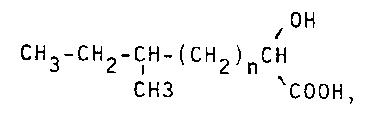 wobei n jeweils eine Zahl von 7 bis 31 darstellt, und
II) Fettsäurepartialglyceriden unverzweigter Fettsäuren,

welche gegebenenfalls in einem geeigneten kosmetischen Träger vorliegen können, zur Bekämpfung grampositiver, insbesondere coryneformer Bakterien, beziehungsweise die Verwendung von Gemischen aus
I) Wollwachssäuren oder Wollwachssäurekomponenten und
II) Fettsäurepartialglyceriden unverzweigter Fettsäuren,

insbesondere Gemischen aus
I)
   - a-Hydroxycarbonsäuren, gemäß der Formel 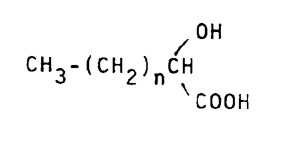 und/oder
   a-Hydroxy-isocarbonsäuren, gemäß der Formel 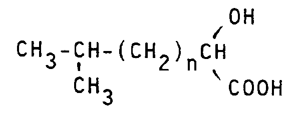 und/oder
   a-Hydroxy-anteisocarbonsäuren, gemäß der Formel 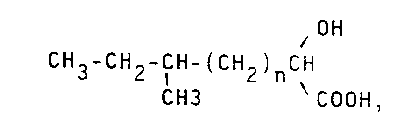 wobei n jeweils eine Zahl von 7 bis 31 darstellt, und
11) Fettsäurepartialglyceriden unverzweigter Fettsäuren,

welche gegebenenfalls in einem geeigneten kosmetischen Träger vorliegen können, zur Verhinderung des Wachstums grampositiver, insbesondere coryneformer Bakterien.

Die erfindungsgemäßen kosmetischen Desodorantien sind besonders vorteilhaft dadurch gekennzeichnet, daß die Wollwachssäuren bzw. die Wollwachssäurekomponenten in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen kosmetischen Desodorantien sind ebenfalls besonders vorteilhaft dadurch gekennzeichnet, daß die Fettsäurepartialglyceride unverzweigter Fettsäuren in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Cremes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der erfindinngsgemäßen desodorierenden Zubereitungen können neben Wasser, Ethanol und Isopropanol, Glycerin und Propylenglykol hautpflegende Fett- oder fettähnliche Stoffe, wie Ölsäuredecvlester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z. B. Hydroxyethyl-oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindinngsgemäßen kosmetischen Desodorantien, welche vorteilhaft alsflüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamtzusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den desodorierenden kosmetischen Zubereitungen gemäß der Erfindung, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien (z.B. a-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Der pH-Wert der erfindinngsgemäßen kosmetischen Desodorantien wird bevorzugt so eingestellt, daß die erfindungsgemäßen Säurekomponenten im wesentlichen als Säuren, und nicht als Anionen, vorliegen, also bevorzugt im sauren bis neutralen Bereich, insbesondere im pH-Bereich von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Zusammensetzungen in Pudersprays eingearbeitet werden, so können die Suspensionsgrundlagen dafür vorteilhaft gewählt werden aus der Gruppe

Kieselsäuregele (z.B. solche die unter dem Handelsnamen Aerosil^{R} erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die angagebenen Zahlenwerte beziehen sich stets auf Gew.-%, sofern nicht ausdrücklich etwas Anderes vermerkt wird. In den Beispielen bedeutet der Begriff "WWS" eine Wollwachssäurefraktion, welche gewonnen wurde aus Rohwollwachssäure durch Kurzwegdestillation im bei 10-¹ bar aus dem Destillationstemperaturintervall von 150 - 200° C. Der Anteil an a-Hydroxycarbonsäuren beträgt dabei ca. 22 - 27 %

### Beispiel 1

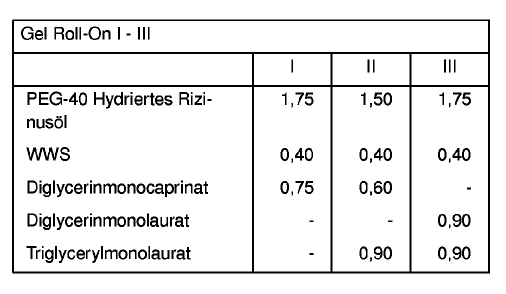

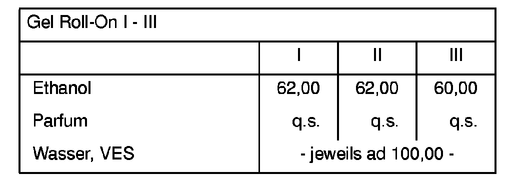

### Beispiel 2

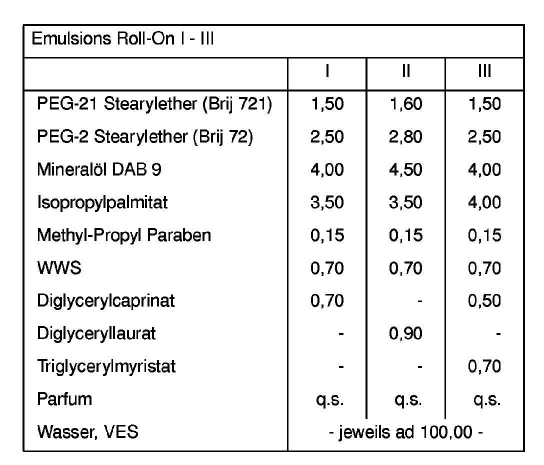

### Beispiel 3

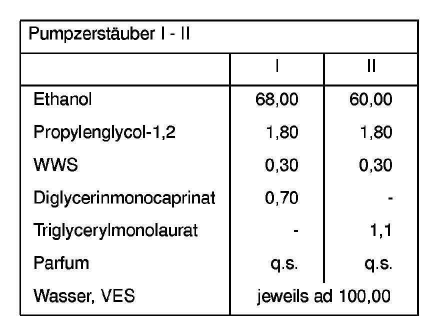

### Beispiel 4

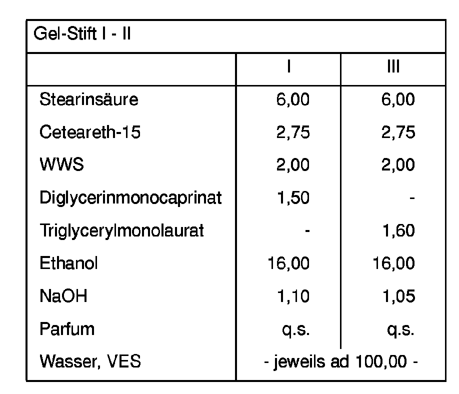

### Beispiel 5

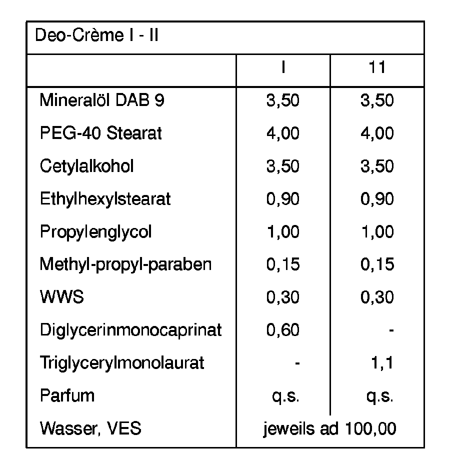

## Revendications

1. Kosmetische Desodorantien, enthaltend Gemische aus
I) Wollwachssäuren oder Wollwachssäurekomponenten und
II) Fettsäurepartialglyceriden unverzweigter Fettsäuren, gewählt aus der Gruppe der
(1) Monocarbonsäure-monoester des Diglycerins der Strukturen 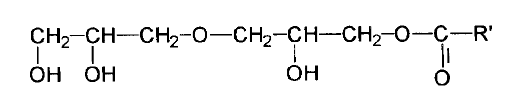 wobei R' einen unverzweigten Alkylrest von 5 bis 17 C-Atomen darstellt,
(2) Monocarbonsäure-monoester des Triglycerins der Strukturen 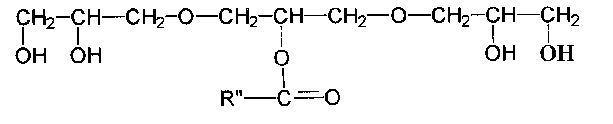 wobei R" einen unverzweigten Alkylrest von 5 bis 17 C-Atomen darstellt.

2. Kosmetische Desodorantien nach Anspruch 1, durch einen Gehalt an II) einer oder mehreren Substanzen gewählt aus der Gruppe
Diglycerinmonocaprinat (DMC) ,
Triglycerinmonolaurat (TML) ,
Diglycerinmonolaurat (DML) ,
Triglycerinmonomyristat (TMM)
gekennzeichnet.

3. Kosmetische Desodorantien nach Anspruch 1, dadurch gekennzeichnet, daß die Wollwachssäurekomponenten gewählt werden aus der Gruppe der
(a) gesättigten unsubstituierte Carbonsäuren, gemäß der Formel 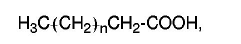
(b) a-Hydroxycarbonsäuren, gemäß der Formel 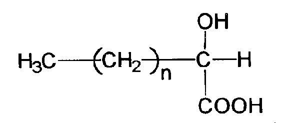
(c) ω-Hydroxycarbonsäuren, gemäß der Formel 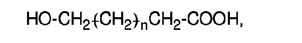
(d) Isocarbonsäuren, gemäß der Formel 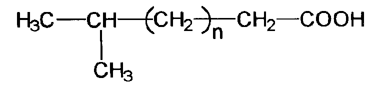
(e) - a-Hydroxy-isocarbonsäuren, gemäß der Formel 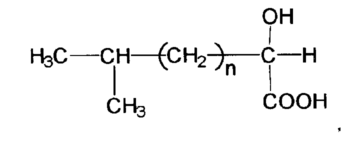
(f) ω-Hydroxy-isocarbonsäuren, gemäß der Formel 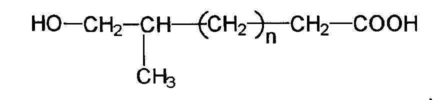
(g) Anteisocarbonsäuren, gemäß der Formel 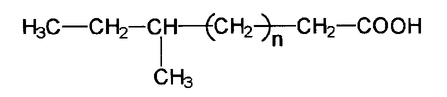
(h) a-Hydroxy-anteisocarbonsäuren, gemäß der Formel 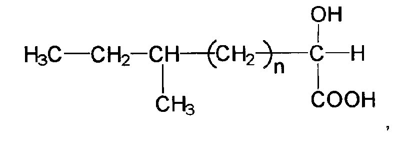
(i) ω-Hydroxy-anteisocarbonsäuren, gemäß der Formel 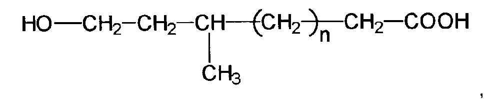 wobei jeweils n Werte von 7 - 31 annimmt.

4. Kosmetische Desodorantien nach Anspruch 1, dadurch gekennzeichnet, daß die Wollwachssäuren bzw. die Wollwachssäurekomponenten in Konzentrationen von 0,05 - 10,00 Gew.-%, bevorzugt 0,1 - 5,0 Gew.-%, vorliegen, jeweils bezogen auf das Gesamtgewicht der Zubereitungen.

5. Kosmetische Desodorantien nach Anspruch 1, dadurch gekennzeichnet, daß die Fettsäurepartialglyceride unverzweigter Fettsäuren in Konzentrationen von 0,05 - 10,00 Gew.-%, bevorzugt 0,1 - 5,0 Gew.-%, vorliegen, jeweils bezogen auf das Gesamtgewicht der Zubereitungen.

6. Kosmetische Desodorantien nach Anspruch 1, dadurch gekennzeichnet, daß sie in Form von
Aerosolen, also aus Aerosolbehältern, Quetschflaschen oder durch eine Pumpvorrichtung versprühbaren Präparaten
- mittels Roll-on-Vorrichtungen auftragbaren flüssigen Zusammensetzungen, Deo-Stiffen (Deo-Sticks)
- aus normalen Flaschen und Behältern auftragbaren W/O- oder O/W-Emulsionen, z.B. Cremes oder Lotionen desodorierenden Tinkturen,
desodorierenden Intimreinigungsmitteln,
desodorierenden Shampoos,
desodorierenden Dusch- oder Badezubereitungen,
desodorierenden Pudern oder
desodorierenden Pudersprays
vorliegen.

7. Verwendung von Gemischen aus
I) Wollwachssäuren oder Wollwachssäurekomponenten und
11) Fettsäurepartialglyceriden unverzweigter Fettsäuren, gewährt aus der Gruppe der
(1) Monocarbonsäure-monoester des Diglycerins der Strukturen 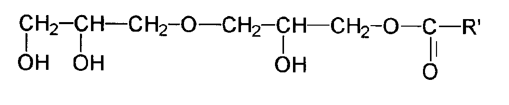 wobei R' einen unverzweigten Alkylrest von 5 bis 17 C-Atomen darstellt,
(2) Monocarbonsäure-monoester des Triglycerins der Strukturen 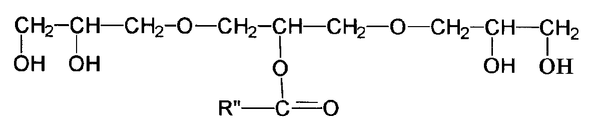 wobei R" einen unverzweigten Alkylrest von 5 bis 17 C-Atomen darstellt,
als desodorierendes Prinzip für kosmetische Desodorantien.

8. Verfahren zur Bekämpfung des durch mikrobielle Zersetzung apokrinen Schweißes hervorgerufenen menschlichen Körpergeruches, dadurch gekennzeichnet, daß eine wirksame Menge an Gemischen aus
I) Wollwachssäuren oder Wollwachssäurekomponenten und
11) Fettsäurepartialglyceriden unverzweigter Fettsäuren, gewählt aus der Gruppe der
(1) Monocarbonsäure-monoester des Diglycerins der Strukturen 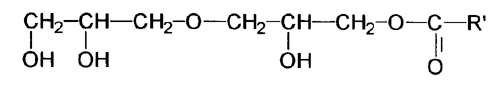 wobei R' einen unverzweigten Alkylrest von 5 bis 17 C-Atomen darstellt,
(2) Monocarbonsäure-monoester des Triglycerins der Strukturen 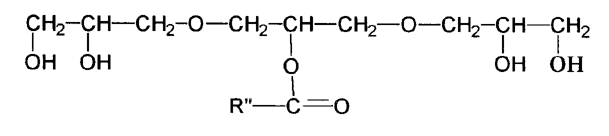 wobei R" einen unverzweigten Alkylrest von 5 bis 17 C-Atomen darstellt,
welche gegebenenfalls in einem geeigneten kosmetischen Träger vorliegen können, auf die Haut aufgetragen wird.

## Claims

1. Cosmetic deodorants containing mixtures of
I) wool wax acids or wool wax acid components, and
II) fatty acid partial glycerides of branched fatty acids, selected from amongst the group of the
(1) diglycerol monocarboxylic acid monoesters of the structures 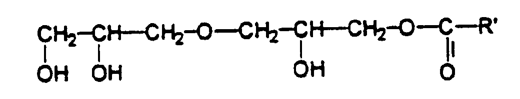 where R' represents an unbranched alkyl radical having 5 to 17 C atoms,
(2) triglycerol monocarboxylic acid monoesters having the structures 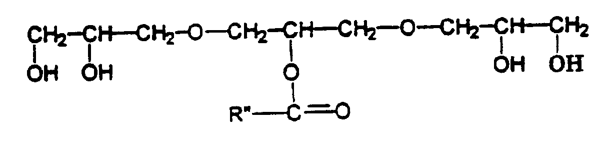 where R" represents an unbranched alkyl radical having 5 to 17 C atoms.

2. Cosmetic deodorants according to Claim 1, characterized in that they contain
II one or more substances selected from amongst the group comprising
diglyceryl monocaprate (DMC),
triglyceryl monolaurate (TML),
diglyceryl monolaurate (DML) and
triglyceryl monomyristate (TMM).

3. Cosmetic deodorants according to Claim 1, characterized in that the wool wax acid components are selected from amongst the group comprising
(a) saturated unsubstituted carboxylic acids of the formula 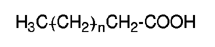
(b) a-hydroxycarboxylic acids of the formula 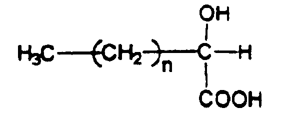
(c) ro-hydroxycarboxylic acids of the formula 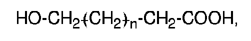
(d) isocarboxylic acids of the formula 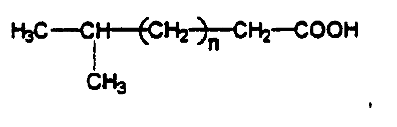
(e) a-hydroxy-isocarboxylic acids of the formula 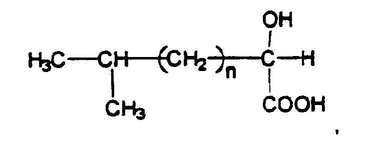
(f) ω-hydroxy-isocarboxylic acids of the formula 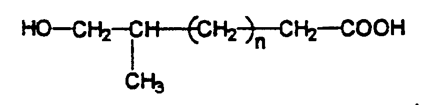
(g) ante-isocarboxylic acids of the formula 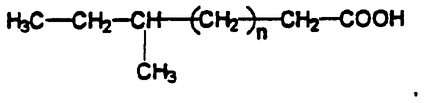
(h) a-hydroxy-ante-isocarboxylic acids of the formula 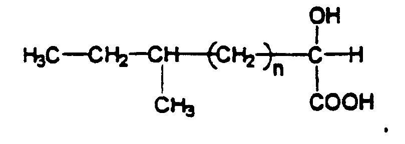
(i) ω-hydroxy-ante-isocarboxylic acids of the formula 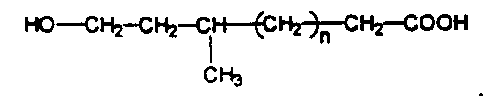
n in each case assuming values from 7-31.

4. Cosmetic deodorants according to Claim 1, characterized in that the wool wax acids or the wool wax acid components are present at concentrations of 0.05-10.00% by weight, preferably 0.1-5.0% by weight, in each case based on the total weight of the preparations.

5. Cosmetic deodorants according to Claim 1, characterized in that the fatty acid partial glycerides of unbranched fatty acids are present at concentrations of 0.05-10.00% by weight, preferably 0.1-5.0% by weight, in each case based on the total weight of the preparations.

6. Cosmetic deodorants according to Claim 1, characterized in that they are in the form of
- aerosols, i.e. preparations which can be sprayed from aerosol containers, squeeze bottles or by means of a pumping device,
- liquid compositions which can be applied by means of roll-on devices,
deodorant sticks,
W/O or O/W emulsions, for example creams or lotions, which can be applied from normal bottles and containers,
deodorizing tinctures,
deodorizing compositions for female hygiene,
deodorizing shampoos,
deodorizing shower or bath preparations,
deodorizing powders or
deodorizing powder sprays.

Use of mixtures of
I) wool wax acids or wool wax acid components, and
II) fatty acid partial glycerides of unbranched fatty acids, selected from amongst the group of the
(1) diglycerol monocarboxylic acid monoesters of the structures 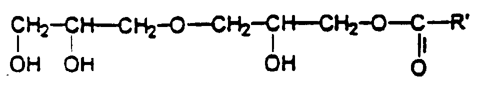 where R' represents an unbranched alkyl radical having 5 to 17 C atoms,
(2) triglycerol monocarboxylic acid monoesters having the structures 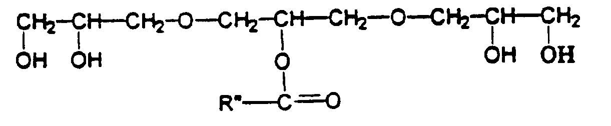 where R" represents an unbranched alkyl radical having 5 to 17 C atoms,
as deodorizing principle for cosmetic deodorants.

8. Method of controlling human body odour caused by microbial decomposition of apocrine sweat, characterized in that an effective amount of mixtures of
I) wool wax acids or wool wax acid components, and
II) fatty acid partial glycerides of unbranched fatty acids, selected from amongst the group of the
(1) diglycerol monocarboxylic acid monoesters of the structures 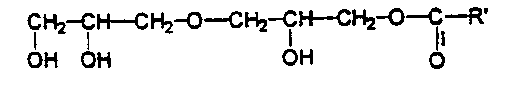 where R' represents an unbranched alkyl radical having 5 to 17 C atoms,
(2) triglycerol monocarboxylic acid monoesters having the structures 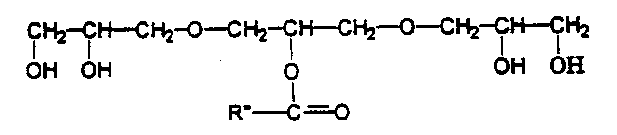 where R" represents an unbranched alkyl radical having 5 to 17 C atoms,
which can optionally be present in a suitable cosmetic carrier, is applied to the skin.

## Revendications

1. Déodorants cosmétiques, contenant des mélanges
I) d'acides de lanoline ou de composants d'acides de lanoline et
II) de glycérides partiels d'acides gras non ramifiés, choisis dans le groupe des
(1) monoesters d'acides monocarboxyliques du diglycérol, de structures 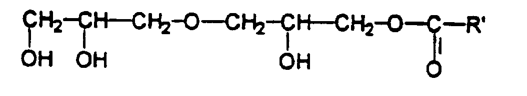 R' représentant un radical alkyle non ramifié ayant de 5 à 17 atomes de carbone,
(2) monoesters d'acide monocarboxylique du triglycérol, de structures 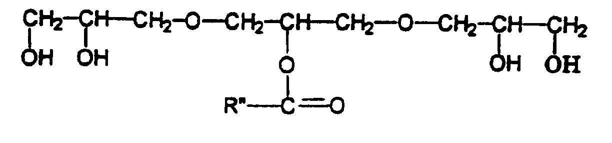 R" représentant un radical alkyle non ramifié ayant de 5 à 17 atomes de carbone.

2. Déodorants cosmétiques selon la revendication 1, caractérisés par une teneur en
II) une ou plusieurs substances choisies dans le groupe constitué par
le monocaprate de diglycérol (DMC),
le monolaurate de triglycérol (TML),
le monolaurate de diglycérol (DML),
le monomyristate de triglycérol (TMM).

3. Déodorants cosmétiques selon la revendication 1, caractérisés en ce que les composants d'acides de lanoline sont choisis dans le groupe constitué par
(a) les acides carboxyliques saturés non substitués, selon la formule 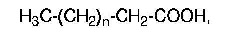
(b) les acides a-hydroxycarboxyliques, selon la formule 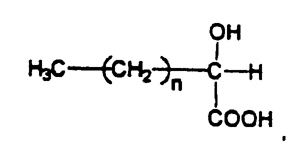
(c) les acides ω-hydroxycarboxyliques, selon la formule 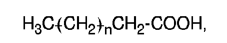
(d) les acides isocarboxyliques, selon la formule 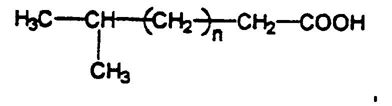
(e) les acides ω-hydroxy-isocarboxyliques, selon la formule 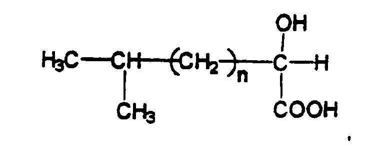
(f) les acides ω-hydroxy-isocarboxyliques, selon la formule 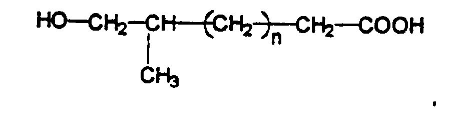
(g) les acides anté-isocarboxyliques, selon la formule 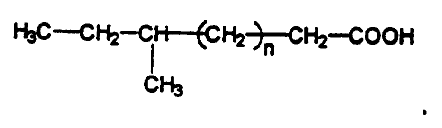
(h) les acides a-hydroxy-anté-isocarboxyliques, selon la formule 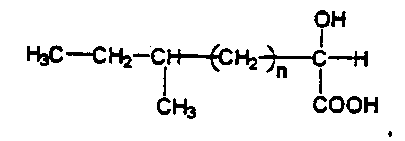
(i) les acides ω-hydroxy-anté-isocarboxyliques, selon la formule 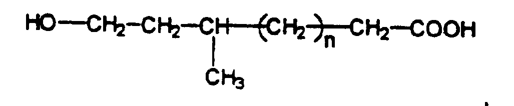
n adoptant dans chaque cas des valeurs allant de 7 à 31.

4. Déodorants cosmétiques selon la revendication 1, caractérisés en ce que les acides de lanoline ou les composants d'acides de lanoline sont présents à des concentrations de 0,05 à 10,00% en poids, de préférence de 0,1 à 5,0% en poids, dans chaque cas par rapport au poids total des préparations.

5. Déodorants cosmétiques selon la revendication 1, caractérisés en ce que les glycérides partiels d'acides gras non ramifiés sont présents à des concentrations de 0,05 à 10,00% en poids, de préférence de 0,1 à 5,0°k en poids, dans chaque cas par rapport au poids total des préparations.

6. Déodorants cosmétiques selon la revendication 1, caractérisés en ce qu'ils se trouvent sous forme
- d'aérosols, c'est-à-dire de préparations pouvant être pulvérisées à partir de contenants pour aérosols, de flacons à presser ou au moyen d'un dispositif à pompe,
- de compositions liquides applicables au moyen d'applicateurs à bille,
- de bâtons déodorants (Deo-sticks),
d'émulsions E/H ou H/E, par exemple de crèmes ou de lotions, pouvant être appliquées à partir de récipients ou de flacons ordinaires,
- de teintures déodorantes,
de produits d'hygiène intime déodorants,
de shampooings déodorants,
de compositions déodorantes pour la douche ou le bain,
de poudres déodorantes ou
de sprays poudres déodorantes.

7. Utilisation de mélanges
II) d'acides de lanoline ou de composants d'acides de lanoline
II) de glycérides partiels d'acides gras non ramifiés, choisis dans le groupe des
(1) monoesters d'acide monocarboxylique du diglycérol, de structures 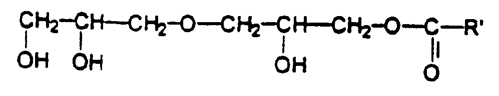 R' représentant un radical alkyle non ramifié ayant de 5 à 17 atomes de carbone,
(2) monoesters d'acide monocarboxylique du triglycérol, de structures 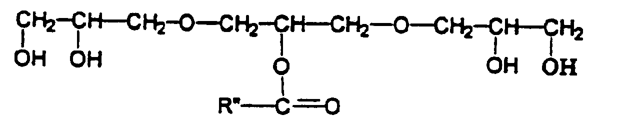 R" représentant un radical alkyle non ramifié ayant de 5 à 17 atomes de carbone,
en tant que principe déodorant pour des déodorants cosmétiques.

8. Procédé pour la lutte contre l'odeur corporelle humaine provoquée par la décomposition microbienne de la sueur apocrine, caractérisé en ce que l'on applique sur la peau une quantité efficace de mélanges
I) d'acides de lanoline ou de composants d'acides de lanoline
II) de glycérides partiels d'acides gras non ramifiés, choisis dans le groupe des
(1) monoesters d'acide monocarboxylique du diglycérol, de structures 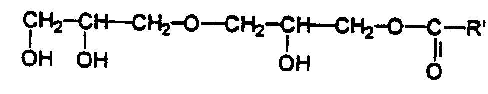 R' représentant un radical alkyle non ramifié ayant de 5 à 17 atomes de carbone,
(2) monoesters d'acide monocarboxylique du triglycérol, de structures 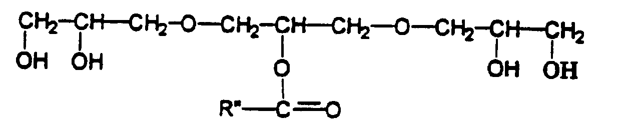 R" représentant un radical alkyle non ramifié ayant de 5 à 17 atomes de carbone,
qui peuvent éventuellement se trouver dans un véhicule cosmétique approprié.
